(19) Europäisches Patentamt
European Patent Office
Office européen des brevets

(11) **EP 3 366 713 B1**

(12) **EUROPÄISCHE PATENTSCHRIFT**

(45) Veröffentlichungstag und Bekanntmachung des Hinweises auf die Patenterteilung:
**01.04.2020 Patentblatt 2020/14**

(51) Int Cl.:
***C07D 307/48*** *(2006.01)*

(21) Anmeldenummer: **17158248.9**

(22) Anmeldetag: **27.02.2017**

(54) **VERFAHREN ZUR HERSTELLUNG VON HOLZVERBUNDWERKSTOFFEN**

METHOD FOR PRODUCTION OF WOOD COMPOSITE PRODUCTS

PROCÉDÉ DE FABRICATION DE MATIÈRES COMPOSITES À BASE DE BOIS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**29.08.2018 Patentblatt 2018/35**

(73) Patentinhaber: **AVALON Industries AG**
**6304 Zug (CH)**

(72) Erfinder:
• **BADOUX, Francois**
  **6314 Unterägeri (CH)**
• **KRAWIELITZKI, Stefan**
  **6343 Holzhäusern (CH)**
• **MORTATO, Mariangela**
  **4051 Basel Stadt (CH)**
• **FREI, Reto**
  **2533 Evilard (CH)**
• **LAGEL, Marie-Christine**
  **67670 Mommenheim (CH)**
• **HOLMES, Christopher**
  **4208 Nunningen (CH)**

(74) Vertreter: **Geitz Truckenmüller Lucht Christ Patentanwälte PartGmbB**
**Obere Wässere 3-7**
**72764 Reutlingen (DE)**

(56) Entgegenhaltungen:
**US-A- 2 776 948        US-A- 4 524 164**
**US-A1- 2013 345 450**

**Beschreibung**

Die vorliegende Erfindung betrifft ein Verfahren zur Herstellung von Holzverbundwerkstoffen. Im Besonderen betrifft die Erfindung ein Verfahren zur Herstellung von Holzverbundwerkstoffen enthaltend die Schritte:

- Bereitstellen eines thermisch härtbaren Harzes, indem man eine polykondensationsfähige phenolische Verbindung und/oder einen Aminoplastbildner mit 5-Hydroxymethylfurfural (HMF) unter zur Bildung von Polykondensationsprodukten führenden Bedingungen umsetzt,
- Inkontaktbringen des Harzes mit lignocellulosehaltigem Material, und
- Aushärten des Harzes unter Bildung des Holzverbundwerkstoffs.

Holzverbundwerkstoffe werden typischerweise aus lignocellulosehaltigem Material wie Holzspänen, Holzfasern oder Holzchips und einem thermisch härtbaren Harz hergestellt. Beispielhaft für thermisch härtbare Harze seien Aminoharze mit den Aminoplastbildnern Harnstoff, Melamin und Dicyandiamid, Phenol-Harze oder Amino-Phenol-Harze genannt. Die Holzverbundwerkstoffe werden üblicherweise erhalten, indem man das lignocellulosehaltige Material mit den Harzen in Kontakt bringt und bei erhöhten Temperaturen verpresst, wobei die Harze ausgehärtet werden, was mit einer Vernetzung einhergeht.

Die US 4 524 164 A betrifft ein formaldehydfreies thermisch härtbares Klebharz und ein Verfahren zur Verwendung des Klebharzes für die Bindung von lignocellulosehaltigem Material, um Produkte wie Sperrholz und Spanplatten zu formen. Zunächst wird ein flüssiges schmelzbares Harz hergestellt, indem eine wässrige Zucker- oder Stärkelösung in Gegenwart eines Vernetzungsmittels, welches ausgewählt ist aus Harnstoff oder einem Phenol oder Mischungen aus diesen, mit einer anorganischen Säure oder ihrem Ammoniumsalz und einem Metallionen-Katalysator erhitzt wird. Das schmelzbare Harz wird anschließend mit einem organischen Säureanhydrid gemischt und auf die Oberfläche des lignocellulosehaltigen Materials aufgetragen. Zur Formung von Produkten wie Sperrholz oder Spanplatten wird das Gemisch sodann Hitze und Druck ausgesetzt.

Die mit den Harzen erzeugten Holzverbundwerkstoffe finden ihre praktische Anwendung aufgrund ihrer guten mechanischen Eigenschaften wie eine gute Innere Bindung (IB) und Unempfindlichkeit gegenüber Feuchtigkeit, vor allem gegenüber Wasserdampf, insbesondere im Möbelbau, wo sie beispielsweise in Form von Sperrholz-, Faserverbund-, Span- und Mehrschichtplatten eingesetzt werden. Dort werden sie überwiegend in Innenräumen verwendet. Aus diesem Grund ist es wichtig, dass die Holzverbundwerkstoffe keine schädlichen Verbindungen emittieren. Die schädlichen Verbindungen stammen dabei hauptsächlich aus den verwendeten Harzen.

Thermisch härtbare Harze werden in der Regel durch die Polykondensation von phenolischen Verbindungen und/oder Aminoplastbildnern mit reaktiven Carbonylverbindungen, insbesondere Aldehyden, gewonnen. Aufgrund seiner hohen Reaktivität kommt für die Polykondensation vorwiegend das als gesundheitsgefährdend einzustufende Formaldehyd zum Einsatz. Zur Förderung der Umsetzung wird häufig mit einem Überschuss an Formaldehyd gearbeitet, sodass die Harze einen hohen Gehalt an freiem, leicht flüchtigem Formaldehyd aufweisen. Die Formaldehydemission der Harze und der mit diesen hergestellten Holzverbundwerkstoffen ist daher ebenfalls hoch.

Aufgrund des Gefährdungspotenzials ist man seit Jahren darum bemüht, den Gehalt an Formaldehyd zu reduzieren. Eine Maßnahme hierbei ist, Formaldehyd bei der Herstellung der Harze durch andere reaktive Verbindungen zu ersetzen. 5-Hydroxymethylfurfural (HMF) wurde bereits als ein vielversprechender Kandidat hierfür identifiziert, da dieses eine Fähigkeit besitzt, quervernetzende Bindungen auszubilden, schwer-flüchtig sowie praktisch ungiftig ist und aus erneuerbaren Rohstoffen gewonnen werden kann.

In der Fachzeitschrift European Journal of Wood Products wird ein HMFmodifiziertes Harnstoff-Formaldehyd-Harz beschrieben, bei dessen Herstellung bis zu etwa 30 Gew.% des Formaldehyds durch gereinigtes, kristallines HMF ersetzt wurden (N. Esmaeili et al., DOI 10.1007/s0017-016-1072-8). Mit diesem Harz hergestellte Spanplatten zeigen eine innere Bindung (IB) von $\geq 0{,}35$ N/mm$^2$, wie sie zur Erfüllung des Mindeststandards für Platten in Innenräumen gemäß der europäischen Norm NEN EN 319 derzeit benötigt werden. Nachteilig ist jedoch, dass das Harz und daraus hergestellte Spanplatten noch erhebliche Mengen an toxischem Formaldehyd enthalten.

Die Aufgabe der vorliegenden Erfindung besteht demnach in der Beseitigung der oben genannten Nachteile.

Dies gelingt nach einem ersten Aspekt der vorliegenden Erfindung durch ein Verfahren zur Herstellung von Holzverbundwerkstoffen gemäß den Merkmalen des Anspruchs 1.

Das Verfahren zur Herstellung von Holzverbundwerkstoffen enthält die Schritte:

- Bereitstellen eines thermisch härtbaren Harzes, indem man eine polykondensationsfähige phenolische Verbindung und/oder einen Aminoplastbildner mit 5-Hydroxymethylfurfural (HMF) unter zur Bildung von Polykondensationsprodukten führenden Bedingungen umsetzt,
- Inkontaktbringen des Harzes mit lignocellulosehaltigem Material, und
- Aushärten des Harzes unter Bildung des Holzverbundwerkstoffs.

Das Verfahren zeichnet sich dadurch aus, dass das 5-Hydroxymethylfurfural mindestens ein HMF-Oligomer umfasst.

Es wurde gefunden, dass zur Herstellung von Holzverbundwerkstoffen auf Formaldehyd verzichtet werden kann, sofern für die Polykondensation im Bereitstellungsschritt HMF eingesetzt wird, das HMF-Oligomere enthält. Es wird angenommen, dass die HMF-Oligomere reaktiver sind als HMF-Monomere, was ein Verfahren zur Herstellung von Holzverbundwerkstoffen ohne den Einsatz von Formaldehyd ermöglicht.

Das Auftreten von wasserlöslichen linearen und verzweigten HMF-Oligomeren in Lösungen von HMF ist beispielsweise aus der DE 10 2014 112 240 A1 bekannt. Die Bildung der HMF-Oligomere kann beispielsweise mittels HPLC-Analysen verfolgt werden.

Als HMF-Oligomere im Sinne der vorliegenden Erfindung werden im Unterschied zu HMF-Monomeren Verbindungen aus mindestens zwei verknüpften HMF-Einheiten/Monomeren bezeichnet. Üblicherweise versteht man unter HMF-Oligomeren Verbindungen mit einem Molekulargewicht von bis zu 3000 g/mol. Für das Verfahren eignen sich insbesondere HMF-Oligomere mit einem niedrigen Molekulargewicht, die unter den gewählten Umsetzungsbedingungen in dem gewählten Lösungsmittel löslich oder zumindest dispergiert vorliegen. Der Übergang zwischen gelöster und dispergierter Form kann hierbei fließend sein, sodass in der vorliegenden Erfindung diesbezüglich nicht unterschieden werden soll.

Die Polykondensation zur Bereitstellung des thermisch härtbaren Harzes nimmt man in an sich bekannter Weise vor. Für die Umsetzung geeignete Lösungsmittel sowie geeignete Reaktionsbedingungen wie beispielsweise Reaktionstemperatur und pH-Wert sind dem Fachmann grundsätzlich bekannt. Bevorzugt wird die Umsetzung in einem wässrigen Lösungsmittel durchgeführt.

Für den Fachmann ist es dabei selbstverständlich, dass das mindestens eine HMF-Oligomer in einer Mischung aus HMF-Oligomeren verschiedener Länge und/oder verschiedenen Vernetzungsgrads vorliegen kann. Es ist zudem möglich, durch die Auswahl eines HMF-Oligomers oder durch die Auswahl einer Kombination unterschiedlicher HMF-Oligomere, die Eigenschaften des aus dem Bereitstellungsschritt resultierenden Harzes gezielt auf den technischen Verwendungszweck abzustimmen.

Nach einer vorteilhaften Ausgestaltung des Verfahrens wird die Umsetzung für die Bereitstellung der Harze bei Temperaturen im Bereich von 40°C bis 140°C, bevorzugt im Bereich von 50 bis 140°C, weiter bevorzugt im Bereich von 60°C bis 100°C, besonders bevorzugt im Bereich von 80°C bis 100°C durchgeführt. Prinzipiell kann die Temperatur zur Durchführung der Polykondensation in einem breiten Bereich variiert werden. Es wurde jedoch beobachtet, dass durch die Umsetzung bei höheren Temperaturen reaktivere Harze gewonnen werden können. Besonders hoch reaktive Harze, welche bei der anschließenden Bildung der Holzwerkstoffe geringe Presszeiten zum Aushärten benötigen, können bei Temperaturen im Bereich von 80°C bis 100°C gewonnen werden. Dies war unerwartet, da man bislang davon ausging, dass bereits ab Temperaturen über 50°C eine zunehmende Zersetzung von HMF stattfindet.

Nach einer weiteren vorteilhaften Ausgestaltung des Verfahrens beträgt das molare Verhältnis der eingesetzten HMF-Menge zu der Gesamtmenge an phenolischer Verbindung und/oder Aminoplastbildner 0,20:1 bis 3:1, bevorzugt beträgt das molare Verhältnis 0,3:1 bis 1:1, besonders bevorzugt beträgt das molare Verhältnis 0,45:1 bis 0,70:1. Prinzipiell kann das molare Verhältnis der eingesetzten HMF-Menge zu der Gesamtmenge an phenolischer Verbindung und/oder Aminoplastbildner über einen breiten Bereich variiert werden. Auch ein molarer Überschuss von HMF kann vorteilhaft sein. Ein für die jeweilige phenolische Verbindung, den jeweiligen Aminoplastbildner oder auf ein Gemisch aus phenolischer Verbindung und Aminoplastbildner geeignetes molares Verhältnis lässt sich für den Fachmann leicht ermitteln.

Nach einer weiteren vorteilhaften Ausgestaltung des Verfahrens beträgt der Anteil an HMF-Oligomer 0,05 Gew.% bis 10 Gew.%, bezogen auf die Gesamtmenge an eingesetztem HMF, bevorzugt beträgt der Anteil an HMF-Oligomer 0,1 Gew.% bis 8 Gew.%, bezogen auf die Gesamtmenge an eingesetztem HMF, besonders bevorzugt beträgt der Anteil an HMF-Oligomer 2 Gew.% bis 4 Gew.%, bezogen auf die Gesamtmenge an eingesetztem HMF. Aufgrund der hohen Reaktivität reichen bereits geringe Mengen an HMF-Oligomer aus, um reaktive Harze bereitzustellen. Es ist für den Fachmann selbstverständlich, dass auch höhere Anteile an HMF-Oligomer eingesetzt werden können. Ebenfalls von der Erfindung umfasst ist, dass das HMF-Oligomer bis zu oder bis nahezu 100 Gew.%, bezogen auf die Gesamtmenge an eingesetztem HMF, ausmacht.

Nach einer weiteren vorteilhaften Ausgestaltung des Verfahrens weist das HMF-Oligomer 2 bis 20 Einheiten auf, bevorzugt 2 bis 10 Einheiten, besonders bevorzugt 2 bis 4 Einheiten. HMF-Oligomere mit 2 bis 10 Einheiten sind unter moderaten Bedingungen, das bedeutet bei Raumtemperatur und unter Normaldruck, gut wasserlöslich, sodass die HMF-Oligomere problemlos für eine Polykondensation in einem wässrigen Medium eingesetzt werden können. HMF-Oligomere mit 2 bis 4 Einheiten weisen eine verbesserte Wasserlöslichkeit auf. HMF-Oligomere mit 2 Einheiten sind besonders gut wasserlöslich und eignen sich damit besonders gut für die Umsetzung.

Nach einer weiteren bevorzugten Ausgestaltung des Verfahrens ist das für die Umsetzung eingesetzte HMF-Oligomer ein kohlenstoffverknüpftes HMF-Oligomer. HMF-Oligomere werden im Sinne der vorliegenden Erfindung als kohlenstoffverknüpfte HMF-Oligomere bezeichnet, sofern zumindest zwei HMF-Einheiten über eine Kohlenstoff-Kohlenstoff-Bindung unter Einbeziehung eines aromatisch gebundenen Kohlenstoffatoms an Position 3 oder 4 des Furanrings einer der beiden HMF-Einheiten verknüpft sind. Insbesondere enthält das kohlenstoffverknüpfte HMF-Oligomer zumindest eine erste Einheit, deren Aldehydgruppen-Kohlenstoffatom mit einem aromatisch gebundenen Kohlenstoffatom des Furanrings einer zweiten Einheit verknüpft ist.

Die Erfinder haben herausgefunden, dass sich neben HMF-Oligomeren, die aus der Verknüpfung von Aldehyd- und/oder Hydroxylgruppen der HMF-Einheiten resultieren und die die entsprechenden Ether-, Hemiacetal- und/oder Acetal-Bindungen aufweisen, sowohl unter sauren als auch unter basischen Bedingungen HMF-Oligomere bilden, bei denen Einheiten über eine Kohlenstoff-Kohlenstoff-Bindung verknüpft sind. Diese Bindungen können beispielsweise bei einem elektrophilen Angriff einer Aldehydgruppe eines ersten HMF-Monomers oder einer HMF-Einheit eines HMF-Oligomers auf das Kohlenstoffatom in Position 3 oder 4 eines Furanrings eines zweiten HMF-Monomers oder einer HMF-Einheit eines HMF-Oligomers entstehen.

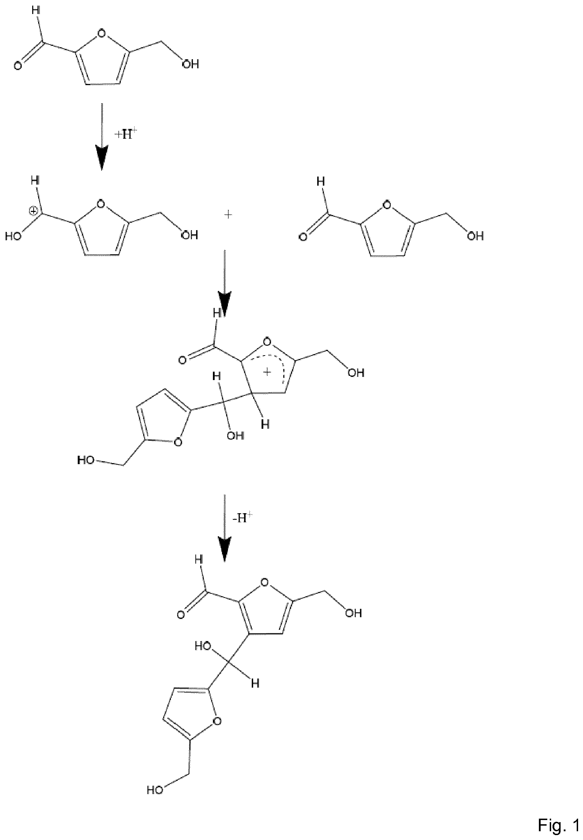

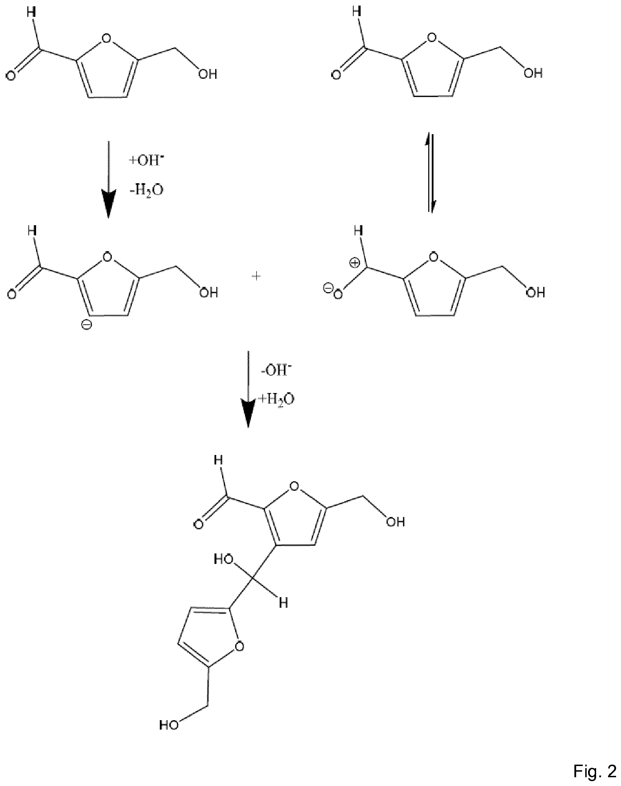

Die für die HMF-Oligomerbildung im Sauren und im Basischen vorgeschlagenen Mechanismen können den Figuren 1 und 2 entnommen werden. Aus diesen wird unter anderem ersichtlich, dass HMF-Oligomere, die über eine Verknüpfung durch eine Kohlenstoff-Kohlenstoff-Bindung verfügen, gleichzeitig auch über mehr freie funktionelle Aldehyd- und/oder Hydroxyl-Gruppen verfügen als HMF-Oligomere, bei denen die Verbindung lediglich über Aldehyd- und/ Hydroxylgruppen des HMFs zustande kommt. Hierdurch ergeben sich sehr reaktive HMF-Oligomere, die über zusätzliche Vernetzungsmöglichkeiten verfügen.

In dem kohlenstoffverknüpften HMF-Oligomer können neben der unter Einbeziehung eines aromatisch gebundenen Kohlenstoffs geknüpften Bindung auch andere Bindungen wie Ether-, Hemiacetal- und/oder Acetal-Bindungen enthalten sein.

Zur Erhöhung der Reaktivität des resultierenden Harzes ist es ausreichend, wenn bereits zwei der HMF-Einheiten unter Einbeziehung eines aromatisch gebundenen Kohlenstoffatoms verknüpft sind. Insbesondere enthalten kohlenstoffverknüpfte HMF-Oligomere mit 2 Einheiten einen vergleichsweise hohen Anteil an freien funktionellen, reaktiven Gruppen pro HMF-Oligomer. Das kohlenstoffverknüpfte HMF-Oligomer kann auch mehrere solcher Kohlenstoff-Kohlenstoff-Verknüpfungen aufweisen.

Ferner können neben den kohlenstoffverknüpften HMF-Oligomeren noch weitere HMF-Oligomere mit Ether-, Hemiacetal-, und/oder Acetal-Bindungen enthalten sein. Aufgrund des hohen Anteils an freien funktionellen Gruppen reichen bereits geringe Mengen an kohlenstoffverknüpftem HMF-Oligomer aus, um sehr reaktive Oligomere bereitzustellen. Ebenfalls von der Erfindung umfasst ist, dass das kohlenstoffverknüpfte HMF-Oligomer bis zu oder bis nahezu 100 Gew.%, bezogen auf die Gesamtmenge an HMF-Oligpmer, ausmacht.

Bei der polykondensationsfähigen phenolischen Verbindung und/oder dem Aminoplastbildner kann es sich um solche handeln, die üblicherweise für die Herstellung von thermisch härtbaren Harzen verwendet werden.

Als polykondensationsfähige phenolische Verbindung kommen hierbei prinzipiell alle hydroxylgruppentragenden aromatischen Verbindungen in Frage, die im Aromaten mindestens ein Kohlenstoffatom aufweisen, das für eine nukleophile Additionsreaktion zwischen phenolischer Verbindung und dem HMF geeignet ist.

Vorteilhafterweise ist die polykondensationsfähige phenolische Verbindung Phenol, Lignin, eine aus Lignin abgeleitete phenolische Verbindung, Resorcin, Hydrochinon, Hydroxyhydrochinon, Brenzcatechin, Phloroglucin oder ein Gemisch von mindestens zwei dieser Verbindungen.

Vorteilhafterweise ist der Aminoplastbildner Harnstoff, Melamin, substituiertes Melamin, substituierter Harnstoff, Acetylendiharnstoff, Guanidin, Thioharnstoff, Thioharnstoffderivat, Diaminoalkan, Diamidoalkan oder ein Gemisch von mindestens zwei dieser Aminoplastbildner.

Dabei können neben den genannten Komponenten auch noch weitere phenolische Verbindungen und/oder Aminoplastbildner vorhanden sein.

In Abhängigkeit von der phenolischen Verbindung und/oder dem Aminoplastbildner kann der pH-Wert im Bereitstellungsschritt über einen breiten Bereich variieren. Der pH-Wert kann beispielsweise im Bereich von 6 bis 10, bevorzugt im Bereich von 7 bis 8,5 liegen.

Nach einer weiteren vorteilhaften Ausgestaltung des Verfahrens wird der Bereitstellungsschritt in einer Lösung solange durchgeführt, bis die Lösung eine gewünschte Viskosität erreicht hat oder die Reaktion abgeschlossen ist. Bevorzugt wird der Bereitstellungsschritt solange durchgeführt, bis die Lösung eine Viskosität von über 200 mPa.s erreicht hat, besonders bevorzugt bis die Lösung eine Viskosität von über 450 mPa.s erreicht hat.

Vorteilhafterweise werden sehr reaktive thermisch härtbare Harze bereitgestellt, die durch Aushärten in Kontakt mit einem lignocellulosehaltigen Material Holzverbundwerkstoffe mit sehr guten mechanischen Eigenschaften liefern.

Bevorzugt umfasst das thermisch härtbare Harz wenigstens ein durch Polykondensation von phenolischen Verbindungen und/oder Aminoplastbildnern mit 5-Hydroxymethylfurfural (HMF) erhaltenes Polymer, wobei das Polymer ein Polykondensationsprodukt einer phenolischen Verbindung und/oder einem Aminoplastbildner mit einem HMF-Oligomer ist.

Unter dem Begriff Polymer werden im Sinne der vorliegenden Erfindung Produkte der Polykondensation verstanden. Die Polymere sind üblicherweise wasserunlöslich.

Besonders bevorzugt ist das Polymer ein Polykondensationsprodukt einer phenolischen Verbindung und/oder einem Aminoplastbildner mit einem kohlenstoffverknüpften HMF-Oligomer, das zumindest eine erste HMF-Einheit enthält, die mit einem aromatisch gebundenen Kohlenstoff einer zweiten HMF-Einheit verknüpft ist.

Der Feststoffgehalt des im Bereitstellungsschritt erhaltenen Harzes kann über einen breiten Bereich variiert werden. Der Feststoffgehalt beträgt wenigstens 40 Gew.%. Bevorzugt liegt der Feststoffgehalt des Harzes im Bereich von 45 bis 80 Gew.%, besonders bevorzugt zwischen 50 und 70 Gew.%.

Nach einer weiteren vorteilhaften Ausgestaltung des Verfahrens enthält das Verfahren mindestens einen weiteren Schritt, der 5-Hydroxymethylfurfural, welches mindestens ein HMF-Oligomer umfasst, für den Schritt der Bereitstellung des thermisch härtbaren Harzes zur Verfügung stellt.

Bevorzugt enthält der Zur-Verfügung-Stellungs-Schritt, dass man eine mehr oder weniger reine Lösung von HMF-Monomeren und/oder HMF-Oligomeren Bedingungen aussetzt, die zur Bildung von HMF-Oligomeren führen. Die Erfinder haben festgestellt, dass wässrige HMF-Lösungen, die beispielsweise aus kristallinem HMF mit Wasser hergestellt wurden, unter Bildung der HMF-Oligomere altern. Die Menge und das Molekulargewicht der HMF-Oligomere kann hierbei über dem Fachmann geläufige Analysemittel wie HPLC und NMR-Spektroskopie bestimmt werden.

Die Bildung von HMF-Oligomeren unter moderaten Bedingungen, das bedeutet, unter Normaldruck und Raumtemperatur, kann im Bereich von Stunden, Tagen oder Wochen liegen.

Besonders bevorzugt umfassen die Bedingungen, denen die HMF-Lösung ausgesetzt wird, eine Alkalisierung oder Ansäuerung der Lösung. Ebenfalls besonders bevorzugt umfassen die Bedingungen das Erhitzen der Lösung, gegebenenfalls in Kombination mit einer Ansäuerung oder Alkalisierung. Durch Ansäuerung, Alkalisierung und Erhitzen kann der Alterungsprozess beschleunigt werden.

Eine besonders bevorzugte Variante des Zur-Verfügung-Stellungs-Schritts beinhaltet, dass man 5-Hydroxymethylfurfural, welches mindestens ein HMF-Oligomer umfasst, durch Behandlung einer wässrigen Suspension von cellulosehaltiger Biomasse und/oder einer wässrigen Kohlenhydrat-Lösung von mindestens einer Hexose und/oder einer wässrigen 5-Hydroxymethylfurfural-Lösung unter hydrothermalen Bedingungen zur Verfügung stellt.

Die Behandlung von Biomasse wie pflanzlichen Rohstoffen, von Kohlenhydraten oder von aus Kohlenhydraten abgeleiteten Verbindungen unter hydrothermalen Bedingungen zur Gewinnung von 5-HMF ist bekannt und sieht vor, das Ausgangsmaterial in wässrigem Medium Druck und erhöhter Temperatur auszusetzen. Bei der Behandlung einer wässrigen Suspension von cellulosehaltiger Biomasse und/oder einer wässrigen Kohlenhydrat-Lösung von mindestens einer Hexose und/oder einer wässrigen 5-Hydroxymethylfurfural-Lösung unter hydrothermalen Bedingungen werden HMF-Oligomere gebildet.

Cellulosehaltige Biomasse, die häufig als Abfallprodukt der landwirtschaftlichen Erzeuger anfällt, ist aufgrund Ihres geringen Kostenfaktors besonders bevorzugt. Bevorzugte Hexosen sind Fructose oder Glucose, insbesondere kann es sich um Fructose oder Gemische aus Fructose und Glucose handeln.

Bevorzugte hydrothermale Bedingungen sind Sattdampfdruck und Temperaturen von 150 bis 250°C. Dies hat den Vorteil, dass die Bildung von HMF-Oligomeren in Abhängigkeit des Ausgangsmaterials innerhalb von Minuten bis zu wenigen Stunden abgeschlossen ist.

Bevorzugt wird der Zur-Verfügung-Stellungs-Schritt so lange durchgeführt, bis die gewünschte Menge an HMF-Oligomer erreicht ist oder die Reaktion abgeschlossen ist.

Bevorzugt liegt das HMF, welches mindestens ein HMF-Oligomer umfasst, am Ende des Zur-Verfügung-Stellungs-Schritts in wässriger Lösung vor. Weiter bevorzugt ist es, den Gehalt, die Größe und/oder die Konzentration des Oligomers oder der Oligomere zu beeinflussen. Besonders bevorzugt wird der Gehalt des Oligomers oder der Oligomere beeinflusst, indem die im Zur-Verfügung-Stellungs-Schritt erhaltene Lösung einer Filtration an mindestens einem Filtriermittel unterzogen wird. Die Behandlung einer wässrigen HMF-Lösung nach einer hydrothermalen Karbonisierung wird beispielsweise in der DE 10 2014 112 240 A1 beschrieben.

Nach einer weiteren vorteilhaften Ausgestaltung des Verfahrens umfasst das lignocellulosehaltige Material, welches mit dem Harz in Kontakt gebracht wird, Holzspäne, Holzfasern, Holzflocken, Holzchips, Holzteilchen, Holzstreifen, Holzplättchen und -platten sowie Mischungen dieser. Das lignocellulosehaltige Material kann hinsichtlich der Vielzahl an unterschiedlichen Holzverbundwerkstoffen in einem breiten Bereich variieren.

Das Inkontaktbringen des Harzes mit lignocellulosehaltigem Material kann durch dem Fachmann bekannte Methoden erfolgen. Üblicherweise wird in Abhängigkeit der Beschaffenheit des Harzes und der Konfiguration des lignocellulosehaltigen Materials das Inkontaktbringen durchgeführt, beispielsweise durch Aufsprühen, Aufstreichen, Vermischen unter mechanischem Rühren oder Walzenauftrag.

Nach einer weiteren vorteilhaften Ausgestaltung des Verfahrens wird das lignocellulosehaltige Material mit einer Menge von 2 Gew.% bis 20 Gew.%, bevorzugt mit einer Menge von 5 Gew.% bis 15 Gew.%, Harz, bezogen auf das Gewicht des trockenen lignocellulosehaltigen Materials, in Kontakt gebracht. Die Harzmenge kann je nach der Konfiguration des lignocellulosehaltigen Materials und den Anforderungen an den Holzverbundwerkstoff in einem breiten Bereich variieren. Es kann zudem vorteilhaft sein, Mischungen aus zwei oder mehr Harzen mit dem lignocellulosehaltigen Material in Kontakt zu bringen.

Nach einer weiteren vorteilhaften Ausgestaltung des Verfahrens umfasst das Aushärten des Harzes ein Verpressen des lignocellulosehaltigen Materials mit dem Harz. Üblicherweise kommen Drücke von 1 bis 30 mPa zum Einsatz.

Bevorzugt erfolgt das Verpressen bei einer Temperatur im Bereich von 120°C bis 250°C, besonders bevorzugt in dem Bereich von 210°C bis 230°C. Aufgrund der Reaktivität der Harze genügen bereits wenige Minuten zum Erhalt von Holzwerkstoffen mit guten mechanischen Eigenschaften. Bevorzugt liegt die Pressdauer im Bereich von 3 bis 10 Minuten, besonders bevorzugt liegt die Pressdauer im Bereich von 5 bis 8 Minuten. Eine kurze Pressdauer ist sowohl aus produktionstechnischen als auch wirtschaftlichen Gesichtspunkten vorteilhaft.

Gewünschtenfalls kann das Vernetzungsvermögen der Harze dadurch erhöht werden, dass man den Harzen einen Härter zusetzt. Bevorzugt liegt die Menge des Härters im Bereich von 2 Gew.% bis 7 Gew.%, bezogen auf die Harzmenge, besonders bevorzugt im Bereich von 2 Gew.% bis 5,5 Gew.%, bezogen auf die Harzmenge, ganz besonders bevorzugt im Bereich von 2 Gew.% bis 3 Gew.%, bezogen auf die Harzmenge. Härter können insbesondere Hexamethylentetramin oder Ammoniumsalze wie Ammoniumsulfat sein. Die Reaktivität der HMF-Oligomere ist jedoch so hoch, dass lediglich sehr geringe Mengen an Härter eingesetzt werden müssen, um Harze mit einem hohen Vernetzungsvermögen zu erhalten. Es kann auch vollkommen auf einen Härter verzichtet werden.

Die erhaltenen Holzverbundwerkstoffe können schließlich zur Stabilisierung in einem Trockenschrank oder Holztrockner bei Temperaturen im Bereich von 10°C bis 100°C unter kontrollierter Atmosphäre nachbehandelt werden. Eine solche kann beispielsweise eine relative Luftfeuchte im Bereich von 40% bis 70% umfassen.

Ein Vorteil der Herstellung eines Holzverbundwerkstoffs mit dem erfindungsgemäßen Verfahren ist, dass die Holzverbundwerkstoffe formaldehyd-frei und auf Basis natürlicher, erneuerbarer Rohstoffe hergestellt werden können und dabei eine sehr gute Beständigkeit gegen Feuchtigkeit, insbesondere Wasserdampf, aufweisen. Ein weiterer Vorteil des Verfahrens ist, dass aufgrund der Reaktivität der HMF-Oligomere kurze Presszeiten im Minutenbereich ausreichen, um einen Holzverbundwerkstoff mit sehr guten mechanischen Eigenschaften zu erhalten. Dies ist unter wirtschaftlichen Gesichtspunkten und hinsichtlich der industriellen Fertigung von Holzverbundwerkstoffen hoch erwünscht.

Die nachfolgenden Beispiele dienen lediglich der Erläuterung der Erfindung und sollen diese in keiner Weise beschränken.

Beispiel 1:

Herstellung von Spanplatten

a) Bereitstellung einer HMF-Lösung mit HMF-Oligomeren:

Eine 16%tige wässrige Lösung aus kristallinem HMF wurde gleichzeitig konzentriert und gealtert, indem sie in einem Rotationsverdampfer bei 45°C und 30 mbar eingeengt wurde, bis die Konzentration an HMF 50 Gew.%, bezogen auf die Lösung betrug.

b) Herstellung und Vergleich der Eigenschaften von Harnstoff-HMF-Harzen:

Es wurden zwei Harze hergestellt, die sich in ihrem molaren Verhältnis von Harnstoff zu HMF unterschieden. Ein erstes Harz wurde mit einem Verhältnis von 1:0,5 Harnstoff zu HMF, im Folgenden mit UH(1:0,5) bezeichnet, hergestellt. Ein zweites Harz wurde mit einem Verhältnis von 1:0,25 Harnstoff zu HMF, im Folgenden mit UH(1:0,25) bezeichnet, hergestellt. Der Feststoffgehalt der Harze lag bei etwa 58%. Für beide Harze wurden 400 ml der 50%itigen HMF-Lösung aus a) verwendet. Bei beiden Harzen wurde der Harnstoff mit HMF bei einem pH-Wert von 2 zunächst für 2,5 Stunden und einer Temperatur von 90°C und anschließend mehrere Stunden bei einer Temperatur von 20°C umgesetzt. Die Änderung der Viskosität der Harze wurde dabei beobachtet.

Tabelle 1: Viskositätszunahme in Abhängigkeit der Zeit

| Zeit [Stunden] | Viskosität [mPa.s] | |
|---|---|---|
| | UH(1:0,5) | UH(1:0,25) |
| 4 | 470 | - |
| 24 | 1275 | 58 |
| 48 | - | 60 |
| 120 | - | 65 |
| 144 | - | 65 |

(fortgesetzt)

| Zeit [Stunden] | Viskosität [mPa.s] | |
|---|---|---|
| | UH(1:0,5) | UH(1:0,25) |
| 168 | - | 65 |

c) Verpressen von Holzspänen zu Spanplatten:

Für das anschließende Verpressen von Holzspänen wurde das Harz UH(1:0,5) mit einer Viskosität von 1275 mPa.s und das Harz UH(1:0,25) mit einer Viskosität von 65 mPa.s verwendet. Die Harze wurden jeweils mit den Holzspänen und mit Hexamethylentetramin vermischt und dann bei 220°C gepresst zur Herstellung von 250 mm x 250 mm x 16 mm großen Platten. Die Beladung des trockenen Holzes betrug 10 Gew.% Harzfeststoff, bezogen auf die Holzmenge. Um den Einfluss verschiedener Presszeiten und verschiedener Mengen an Härter zu testen, wurden mehrere Platten unter Variation der Zeiten und der Mengen an Hexamethylentetramin hergestellt. Die mit den beiden Harzen UH(1:0,5) und UH(1:0,25) für die Spanplatten erhaltenen Werte sind in der Tabelle 2 angegeben.

Zum Vergleich wurde ein drittes Harz, UH45(1:0,5), hergestellt, indem die Komponenten des Harzes UH(1:0,5) bei einer tieferen Temperatur von 45°C umgesetzt wurden. Das Harz UH45(1:0,5) wurde ebenfalls für das Verpressen von Holzspänen zu 250 mm x 250 mm x 16 mm Spanplatten verwendet. Die für diese Spanplatten erhaltenen Werte sind ebenfalls in der Tabelle 2 angegeben.

Der Vergleich der mit den Harzen hergestellten Platten ergab, dass grundsätzlich bei einer längeren Pressdauer bessere Werte für die Innere Festigkeit erhalten werden.

Mit einem Molverhältnis von Harnstoff zu HMF von 1:0,5 erreichen die Platten 3 und 4 die hohen Werte von 52 N/mm$^2$ und 55 N/mm$^2$. Diese Werte sind auf eine Pressdauer von 7,5 min in Verbindung mit einer hohen Herstellungstemperatur der Harze von 90°C zurückzuführen.

Die Platten 1 und 2 sowie 5 und 6 verdeutlichen den Einfluss der Temperatur bei der Herstellung der Harze.

Selbst Platten, welche mit geringeren Mengen an HMF hergestellt wurden, liefern ein befriedigendes Ergebnis, wenn die Pressdauer erhöht wird, wie die Platten 7 bis 10 zeigen.

Hinsichtlich dem Härter hat sich gezeigt, dass sich unterschiedliche Härtermengen weniger stark bis nicht bemerkbar machen, sofern die Platten mit einem gewissen Anteil an HMF hergestellt wurden, wie die Platten 3 bis 6 zeigen. Die Platten 7 und 10 mit niedrigeren Anteilen an HMF werden deutlich stärker von der Härtermenge beeinflusst. Die Werte verdeutlichen, dass infolge der positiven Eigenschaften der verwendeten HMF-Oligomere die benötigten Härtermengen drastisch reduziert werden können, wobei Produkte mit identischer bzw. vergleichbarer Inneren Bindungsstärke erhalten werden können.

Innere Bindungsstärke (IB) gemäß NF EN 319 (AFNOR 1993):
Die innere Bindungsstärke in [N/mm$^2$] wird durch die folgende Formel ausgedrückt:

$$IB = \frac{Fmax}{a x b},$$

wobei *Fmax* die Bruchkraft, a die Breite und b die Länge der Platte ist.

Die NF EN 319 (AFNOR 1993) sieht für Span- und Faserplatten mit einer Stärke im Bereich von 13 mm bis 20 mm eine Innere Bindungsstärke von ≤0,35 N/mm$^2$ vor.

Die Platten zur Untersuchung der Inneren Bindungsstärke wurden durch Zerschneiden aus den unter c) hergestellten Platten gewonnen. Ihre Größe betrug 50 mm x 50 mm. Vor dem Zerschneiden wurden die Platten in einem Trockner bei 20°C und einer relativen Luftfeuchtigkeit von 65% stabilisiert.

Die Platten wurden mit Hilfe eines Heißschmelzklebstoffs auf einer Unterlage befestigt. Die Bestimmung der Inneren Bindungsstärke fand gemäß NF EN 319 (AFNOR 1993) maschinell senkrecht zur Ebene der Platten statt.

Tabelle 2: Parameter der Spanplattenherstellung und Eigenshcaften der Spanplatten

| Platte | Harz | Synthesetemperatur [°C] | Viskosität [mPa.s] | Molverhältnis Harnstoff:HMF | Presstemperatur [°C] | Pressdauer [min] | Härter [%] | Dichte [kg/m³] | Innere Bindungsstärke (IB) [N/mm²] |
|---|---|---|---|---|---|---|---|---|---|
| 1 | UH45 (1: 0,5) | 45 | 382 | 1:0.5 | 220 | 5.5 | 5 | 733 | 0.27 |
| 2 | UH45 (1: 0,5) | 45 | 382 | 1:0.5 | 220 | 5.5 | 2.5 | 729 | 0.21 |
| 3 | UH(1: 0,5) | 90 | 1275 | 1:0.5 | 220 | 7.5 | 5 | 717 | 0.55 |
| 4 | UH(1: 0,5) | 90 | 1275 | 1:0.5 | 220 | 7.5 | 2.5 | 718 | 0.52 |
| 5 | UH(1: 0,5) | 90 | 1275 | 1:0.5 | 220 | 5.5 | 5 | 715 | 0.43 |
| 6 | UH(1: 0,5) | 90 | 1275 | 1:0.5 | 220 | 5.5 | 2.5 | 718 | 0.43 |
| 7 | UH(1: 0,25) | 90 | 65 | 1:0.25 | 220 | 7.5 | 5 | 714 | 0.44 |
| 8 | UH(1: 0,25) | 90 | 65 | 1:0.25 | 220 | 6.5 | 5 | 715 | 0.39 |
| 9 | UH(1: 0,25) | 90 | 65 | 1:0.25 | 220 | 5.5 | 5 | 712 | 0.31 |
| 10 | UH(1: 0,25) | 90 | 65 | 1:0.25 | 220 | 7.5 | 2.5 | 713 | 0.36 |

Weitere Vorteile und vorteilhafte Ausgestaltungen sind den Ansprüchen und der nachfolgenden Zeichnung zu entnehmen.

Es zeigen

Figur 1 einen vorgeschlagenen Mechanismus der Kohlenstoff-Kohlenstoff-Bindungsbildung unter sauren Bedingungen anhand der Dimerisierung zweier HMF-Moleküle, sowie

Figur 2 einen vorgeschlagenen Mechanismus der Kohlenstoff-Kohlenstoff-Bindungsbildung unter basischen Bedingungen anhand der Dimerisierung zweier HMF-Moleküle.

Sämtliche Merkmale der Erfindung können sowohl einzeln als auch in beliebiger Kombination miteinander erfindungswesentlich sein.

**Patentansprüche**

1. Verfahren zur Herstellung von Holzverbundwerkstoffen enthaltend die Schritte:

   - Bereitstellen eines thermisch härtbaren Harzes, indem man eine polykondensationsfähige phenolische Verbindung und/oder einen Aminoplastbildner mit 5-Hydroxymethylfurfural (HMF) unter zur Bildung von Polykondensationsprodukten führenden Bedingungen umsetzt,
   - Inkontaktbringen des Harzes mit lignocellulosehaltigem Material, und
   - Aushärten des Harzes unter Bildung des Holzverbundwerkstoffs,

   **dadurch gekennzeichnet, dass** das 5-Hydroxymethylfurfural mindestens ein HMF-Oligomer umfasst.

2. Verfahren nach Anspruch 1, **dadurch gekennzeichnet, dass** die Umsetzung für die Bereitstellung der Harze bei Temperaturen im Bereich von 40°C bis 140°C durchgeführt wird.

3. Verfahren nach einem der vorhergehenden Ansprüche, **dadurch gekennzeichnet, dass** der Anteil an HMF-Oligomer, bezogen auf die Gesamtmenge an eingesetztem HMF, 0,05 Gew.% bis 10 Gew.% beträgt.

4. Verfahren nach einem der vorhergehenden Ansprüche, **dadurch gekennzeichnet, dass** das HMF-Oligomer 2 bis 20 Einheiten aufweist.

5. Verfahren nach einem der vorhergehenden Ansprüche, **dadurch gekennzeichnet, dass** das HMF-Oligomer ein kohlenstoffverknüpftes HMF-Oligomer ist.

6. Verfahren nach einem der vorhergehenden Ansprüche, **dadurch gekennzeichnet, dass** die polykondensationsfähige phenolische Verbindung Phenol, Lignin, eine aus Lignin abgeleitete phenolische Verbindung, Resorcin, Hydrochinon, Hydroxyhydrochinon, Brenzcatechin, Phloroglucin oder ein Gemisch von mindestens zwei dieser Verbindungen ist.

7. Verfahren nach einem der vorhergehenden Ansprüche, **dadurch gekennzeichnet, dass** der Aminoplastbildner Harnstoff, Melamin, substituiertes Melamin, substituierter Harnstoff, Acetylendiharnstoff, Guanidin, Thioharnstoff, Thioharnstoffderivat, Diaminoalkan, Diamidoalkan oder ein Gemisch von mindestens zwei dieser Aminoplastbildner ist.

8. Verfahren nach einem der vorhergehenden Ansprüche, **dadurch gekennzeichnet, dass** der Bereitstellungsschritt in einer Lösung solange durchgeführt wird, bis die Lösung eine gewünschte Viskosität erreicht hat oder die Reaktion abgeschlossen ist.

9. Verfahren nach einem der vorhergehenden Ansprüche, **dadurch gekennzeichnet, dass** das Verfahren mindestens einen weiteren Schritt enthält, der 5-Hydroxymethylfurfural, welches mindestens ein HMF-Oligomer umfasst, für den Bereitstellungsschritt zur Verfügung stellt.

10. Verfahren nach Anspruch 9, **dadurch gekennzeichnet, dass** das 5-Hydroxymethylfurfural durch Behandlung einer wässrigen Suspension von cellulosehaltiger Biomasse und/oder einer wässrigen Kohlenhydrat-Lösung von mindestens einer Hexose und/oder einer wässrigen 5-Hydroxymethylfurfural-Lösung unter hydrothermalen Bedingungen zur Verfügung gestellt wird.

11. Verfahren nach einem der vorhergehenden Ansprüche, **dadurch gekennzeichnet, dass** das lignocellulosehaltige Material, welches mit dem Harz in Kontakt gebracht wird, Holzspäne, Holzfasern, Holzflocken, Holzchips, Holzteilchen, Holzstreifen, Holzplättchen und -platten sowie Mischungen dieser umfasst.

12. Verfahren nach einem der vorhergehenden Ansprüche, **dadurch gekennzeichnet, dass** das lignocellulosehaltige Material mit einer Menge von 2 Gew.% bis 20 Gew.%, bevorzugt mit einer Menge von 5 Gew.% bis 15 Gew.%, Harz, bezogen auf das Gewicht des trockenen lignocellulosehaltigen Materials, in Kontakt gebracht wird.

13. Verfahren nach einem der vorhergehenden Ansprüche, **dadurch gekennzeichnet, dass** das Aushärten des Harzes ein Verpressen des lignocellulosehaltigen Materials mit dem Harz umfasst.

## Claims

1. A method for producing wood composite materials, including the steps:

   - preparing a thermosetting resin by reacting a polycondensation-capable phenolic compound and/or an aminoplast-forming agent with 5-hydroxymethylfurfural (HMF) to form polycondensation products under conditions leading to the formation of polycondensation products,
   - bringing the resin into contact with lignocellulose material, and
   - curing the resin to form the wood composite material,

   **characterized in that** the 5-hydroxymethylfurfural comprises at least one HMF oligomer.

2. The method according to claim 1, **characterized in that** the reaction for preparation of the resins is carried out at temperatures in the range of 40°C to 140°C.

3. The method according to claim 1, **characterized in that** the proportion of HMF oligomer is 0.05% by weight to 10% by weight, based on the total amount of HMF used.

4. The method according to any one of the preceding claims, **characterized in that** the HMF oligomer has 2 to 20 units.

5. The method according to any one of the preceding claims, **characterized in that** the HMF oligomer is a carbon-linked HMF oligomer.

6. The method according to any one of the preceding claims, **characterized in that** the polycondensation-capable phenolic compound is phenol, lignin, a phenolic compound derived from lignin, resorcinol, hydroquinone, hydroxyquinone, pyrocatechol, phloroglucinol or a mixture of at least two of these compounds.

7. The method according to any one of the preceding claims, **characterized in that** the aminoplast-forming agent is urea, melamine, substituted melamine, substituted urea, acetylenediurea, guanidine, thiourea, thiourea derivative, diaminoalkane, diamidoalkane or a mixture of at least two of these aminoplast-forming agents.

8. The method according to any one of the preceding claims, **characterized in that** the preparation step is carried out in a solution until the solution has attained a desired viscosity or the reaction has ended.

9. The method according to any one of the preceding claims, **characterized in that** the method includes at least one further step, which makes available 5-hydroxymethylfurfural, comprising at least one HMF oligomer, for the preparation step.

10. The method according to claim 9, **characterized in that** the 5-hydroxymethylfurfural is supplied by treatment of an aqueous suspension of cellulosic biomass and/or of an aqueous carbohydrate solution of at least one hexose and/or one aqueous 5-hydroxymethylfurfural solution under hydrothermal conditions.

11. The method according to any one of the preceding claims, **characterized in that** the lignocellulosic material, which is brought into contact with the resin, comprises wood shavings, wood fibers, wood flakes, wood chips, wood particles, wooden strips, wooden disks and wooden plates as well as combinations thereof.

12. The method according to any one of the preceding claims, **characterized in that** the lignocellulosic material is brought into contact with the resin in an amount of 2% by weight to 20% by weight, preferably an amount of 5% by weight to 15% by weight, based on the weight of the dry lignocellulosic material.

13. The method according to any one of the preceding claims, **characterized in that** the curing of the resin comprises pressing the lignocellulosic material with the resin.

**Revendications**

1. Procédé de production de matériaux composites en bois, ledit procédé comprenant les étapes suivantes :

   - produire une résine durcissable thermiquement par réaction d'un composé phénolique apte à la polycondensation et/ou d'un formateur aminoplaste avec du 5-hydroxyméthylfurfural (HMF) dans des conditions conduisant à la formation de produits de polycondensation,
   - mettre la résine en contact avec un matériau lignocellulosique, et
   - faire durcir la résine pour former le matériau composite en bois,

   **caractérisé en ce que** le 5-hydroxyméthylfurfural comprend au moins un oligomère HMF.

2. Procédé selon la revendication 1, **caractérisé en ce que** la réaction de production de résines est réalisée à des températures dans la gamme allant de 40 °C à 140 °C.

3. Procédé selon l'une des revendications précédentes, **caractérisé en ce que** la proportion d'oligomère HMF, par rapport à la quantité totale de HMF utilisée, est de 0,05 % en poids à 10 % en poids.

4. Procédé selon l'une des revendications précédentes, **caractérisé en ce que** l'oligomère HMF comporte de 2 à 20 unités.

5. Procédé selon l'une des revendications précédentes, **caractérisé en ce que** l'oligomère HMF est un oligomère HMF lié au carbone.

6. Procédé selon l'une des revendications précédentes, **caractérisé en ce que** le composé phénolique apte à la polycondensation est le phénol, la lignine, un composé phénolique dérivé de la lignine, le résorcinol, l'hydroquinone, l'hydroxyhydroquinone, la pyrocatéchine, le phloroglucinol ou un mélange d'au moins deux de ces composés.

7. Procédé selon l'une des revendications précédentes, **caractérisé en ce que** le formateur aminoplaste est l'urée, la mélamine, la mélamine substituée, l'urée substituée, l'acétylène-diurée, la guanidine, la thiourée, un dérivé de la thiourée, le diaminoalcane, le diamidoalcane ou un mélange d'au moins deux de ces formateurs aminoplastes.

8. Procédé selon l'une des revendications précédentes, **caractérisé en ce que** l'étape de production est réalisée dans une solution jusqu'à ce que la solution ait atteint une viscosité souhaitée ou que la réaction soit terminée.

9. Procédé selon l'une des revendications précédentes, **caractérisé en ce que** le procédé contient au moins une étape supplémentaire dans laquelle le 5-hydroxyméthylfurfural, qui comprend au moins un oligomère HMF, est disponible pour l'étape de production.

10. Procédé selon la revendication 9, **caractérisé en ce que** le 5-hydroxyméthylfurfural est disponible par traitement d'une suspension aqueuse de biomasse cellulosique et/ou d'une solution aqueuse de glucides d'au moins un hexose et/ou d'une solution aqueuse de 5-hydroxyméthylfurfural dans des conditions hydrothermales.

11. Procédé selon l'une des revendications précédentes, **caractérisé en ce que** le matériau, contenant de la lignocellulose, qui est mis en contact avec la résine comprend des copeaux de bois, des fibres de bois, des flocons de bois, des rognures de bois, des particules de bois, des lamelles de bois, des plaquettes et des plaques de bois et des mélanges de ceux-ci.

**12.** Procédé selon l'une des revendications précédentes, **caractérisé en ce que** le matériau contenant de la lignocellulose est mis en contact avec une quantité de 2 % en poids à 20 % en poids, de préférence avec une quantité de 5 % en poids à 15 % en poids, de résine, rapportée au poids du matériau lignocellulosique.

**13.** Procédé selon l'une des revendications précédentes, **caractérisé en ce que** le durcissement de la résine comprend le pressage du matériau contenant de la lignocellulose avec la résine.

Fig. 1

EP 3 366 713 B1

Fig. 2

14

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4524164 A

- DE 102014112240 A1